# EUROPEAN PATENT APPLICATION

(11) **EP 3 087 912 A1**
(43) Date of publication of application: **02.11.2016**
(21) Application number: 16166267.1
(22) Date of filing: 20.04.2016
(51) Int. Cl.: A61B 5/00, G06F 19/00, H04W 4/00, H04W 12/06

(54) **SECURITY ACCESS SYSTEM USING A BODY AREA NETWORK**

(30) Priority: 01.05.2015 US 201514701953
(71) Applicant: Honeywell International Inc., Morris Plains, NJ 07950 (US)
(72) Inventor: PHAM, Hai D., Morris Plains, NJ New Jersey 07950 (US); FABER, Jared, Morris Plains, NJ New Jersey 07950 (US)
(74) Representative: Houghton, Mark Phillip

(57) **Abstract**

A system receives body area network (BAN) data from a BAN that is associated with a person as the person comes into the vicinity of an entry or access point to a structure or property. The system operates a door, gate, or other barrier at the entry or access point as a function of the BAN data.

## Description

### Technical Field

The present disclosure relates to security access systems, and in an embodiment, but not by way of limitation, a security access system that uses a body area network.

### Background

A body area network (BAN), also referred to as a wireless body area network (WBAN) or a body sensor network (BSN), is a wireless network of computing devices that are associated with a particular human body or a particular person. BAN devices may be embedded inside the body, may be implants, may be surface-mounted on the body in a fixed position (*i.e.,* wearable technology), or may be accompanied devices that humans can carry in different positions, in clothes pockets, by hand, or in various bags (*e.g*., something akin to a mobile phone or other personal communication device). While there is a trend towards the miniaturization of devices, in particular networks consisting of several miniaturized body sensor units (BSUs) together with a single body central unit (BCU), larger-sized smart devices (*e.g*., tablets and pads) and other accompanied devices can play an important role in terms of acting as a data hub, data gateway, and/or providing a user interface to view and manage BAN applications.

WBAN technology uses wireless personal area network (WPAN) technologies to implement communications on, near, and/or around the human body. The term BAN has come to refer to systems where communication is entirely within, on, and/or in the immediate proximity of a human body. A WBAN system can use WPAN wireless technologies as gateways to reach longer ranges. Through gateway devices, it is possible to connect the wearable devices on the human body to the Internet, so that persons such as medical professionals can access patient data online using the Internet independent of the patient location.

### Brief Description of the Drawings

**FIG. 1** is a block diagram illustrating an embodiment of a security system that uses a body area network.
**FIG. 2** is a block diagram illustrating features and operations of a security system that uses a body area network.

### Detailed Description

In the following description, reference is made to the accompanying drawings that form a part hereof, and in which is shown by way of illustration specific embodiments which may be practiced. These embodiments are described in sufficient detail to enable those skilled in the art to practice the invention, and it is to be understood that other embodiments may be utilized and that structural, electrical, and optical changes may be made without departing from the scope of the present invention. The following description of example embodiments is, therefore, not to be taken in a limited sense, and the scope of the present invention is defined by the appended claims.

The rapid growth in physiological sensors, low-power integrated circuits, and wireless communication has enabled a new generation of wireless sensor networks, now used for purposes such as monitoring traffic, crops, infrastructure, and health. The body area network field is an interdisciplinary area that particularly allows inexpensive and continuous health monitoring with real-time updates of medical records through the Internet or other data communications network. A number of intelligent physiological sensors can be embedded in a living body or integrated into a wearable wireless body area network, which can be used for computer-assisted rehabilitation or early detection of medical conditions. This area relies on the feasibility of implanting very small biosensors inside a living body that are comfortable and that don't impair normal activities. The implanted sensors in the body will collect various physiological changes in order to monitor a being's or patient's health status irrespective of the patient's physical location. The information can be transmitted wirelessly to an external processing unit, which can instantly transmit all information in real time to doctors throughout the world. If an emergency is detected, the physicians can immediately inform the patient through the computer system by sending appropriate messages or alarms. While the level of information provided and energy resources capable of powering the sensors are currently limited because the technology is still in its primitive stage, it is being widely researched and once adopted, is expected to be a breakthrough in healthcare, leading to concepts like telemedicine.

Initial applications of BANs are primarily in the healthcare domain, especially for continuous monitoring and logging vital parameters of patients suffering from chronic diseases such as diabetes, asthma, and heart disease. A BAN that is instantiated on a patient can alert medical care professionals, even before the patient experiences a medical condition such as a heart attack, by measuring changes in the patient's vital signs. Similarly, a BAN on a diabetic patient could auto-inject insulin through a pump as soon as the patient's insulin level declines.

A typical BAN or BSN includes vital sign monitoring sensors, a processor, motion detectors (*e.g*., accelerometers) to help identify the location and position of the monitored individual, and some form of communication to transmit vital sign and motion readings to medical practitioners or care givers. Physiological sensors, such as electrocardiographs (ECG) and saturation of peripheral oxygen (SpO₂) sensors, have been developed. Other sensors, such as a blood pressure sensor, an electroencephalograph (EEG) sensor, and a personal digital assistant (PDA) for BSN interface are also potentially applicable.

The present disclosure relates to the use of a BAN in a security access system.

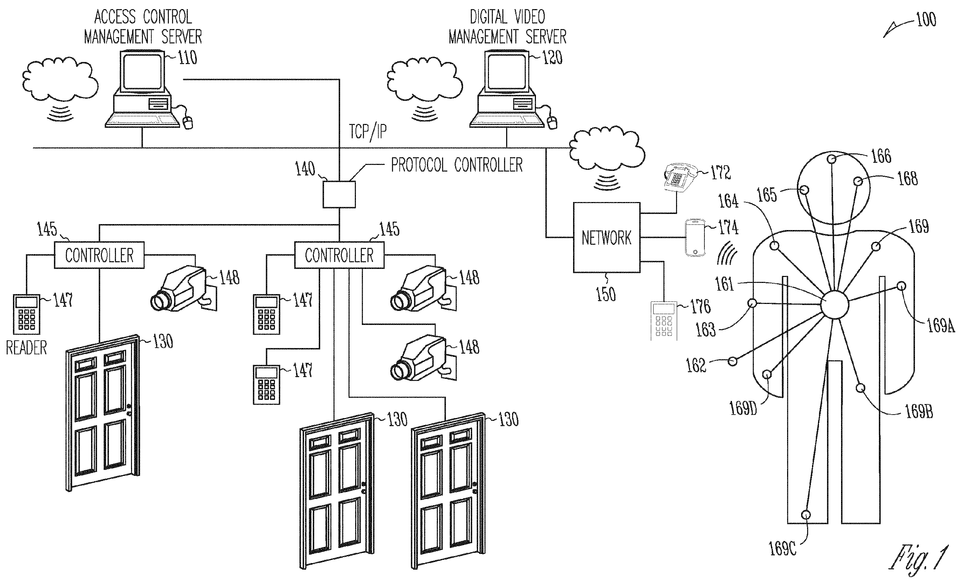

**FIG. 1** is a block diagram of a security access system **100** that uses a body area network. The example system **100** includes an access control management server **110** and a digital video management server **120.** As illustrated in **FIG. 1****,** the access control management server **110** and the digital video management server **120** can be implemented in a cloud environment. The access control management server **110** and the digital video management server **120** are coupled to a security access structure, such as doors or gates **130,** via a protocol converter **140,** a controller **145,** a reader **147,** and a camera or other sensing device **148.** The security access structure **130** can also be associated with access points to pharmaceuticals, chemicals, nutritional provisions, financial instruments, and other valuables in schools, hospitals, laboratories, banks, and other businesses. These access points can include cabinets, safes, closets, rooms, *etc.* Coupled to, and/or part of the security access system **100,** via a network **150,** is the body area network system. The body area network system can be coupled to the network **150** via plain old telephone service (POTS) **172,** cellular service **174,** and/or a wireless local area network (WLAN) **176.**

The body area network includes a processor **161,** and one or more sensors. The sensors can include sensors related to DNA/protein analysis **162,** blood glucose levels **163,** body positioning analysis **164,** vision analysis **165** (including pupil and other eye analyses), EEG sensors **166,** hearing analysis **168,** ECG sensors **169,** blood pressure sensors **169A,** toxin sensors **169B,** implants **169C,** and a means for fingerprint analysis and identification **169D** (*e.g*., storing fingerprint data in a BAN database and using the fingerprint data from the BAN database to identify the person at an access point).

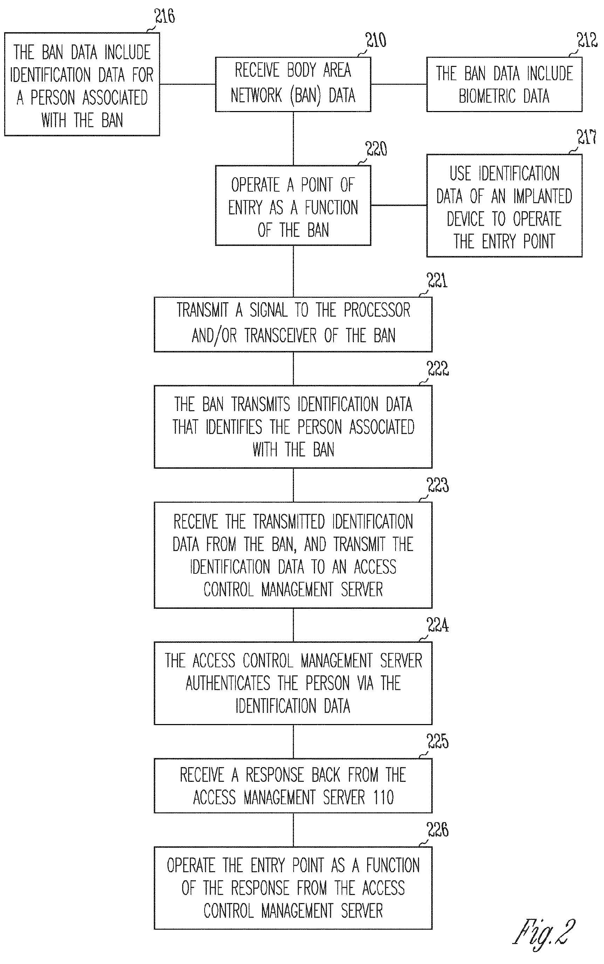

**FIG. 2** is a block diagram illustrating operations and features of processes and systems for using a BAN in a security access system. **FIG. 2** includes a number of process blocks **210** - **226.** Though arranged substantially serially in the example of **FIG. 2****,** other examples may reorder the blocks, omit one or more blocks, and/or execute two or more blocks in parallel using multiple processors or a single processor organized as two or more virtual machines or sub-processors. Moreover, still other examples can implement the blocks as one or more specific interconnected hardware or integrated circuit modules with related control and data signals communicated between and through the modules. Thus, any process flow is applicable to software, firmware, hardware, and hybrid implementations.

Referring to **FIG. 2****,** at **210,** a computer processor receives body area network (BAN) data. As indicated at **12,** the BAN data can include biometric data, and as indicated at **216,** the BAN data can include identification data for a person associated with the BAN. In most instances, the identification data for the person is the same data that is used to identify the person to the computer processor or server that analyses the person's physiological data as sensed by the sensors of the BAN. At **220,** the computer processor operates a point of entry to an access structure as a function of the BAN data. For example, in **FIG. 1****,** the access control management server **110** could, as a function of the received BAN data, transmit a signal through the protocol converter **140** and the controller **145** to open the door **130.** Similarly, the digital video management server **120** could operate a video sensor in the area of the access point as a function of the BAN data. For example, the digital video management server **120** could cause a video sensor to pan an area around the access point as a function of the BAN data, or to begin recording data as a function of the BAN data. As further indicated at **217,** the computer processor can use the identification data (such as a serial number) of an implanted device to identify the person and operate an entry point to an access structure or other property.

The operation **220** of the point of entry of the access structure is further detailed as follows. At **221,** the reader **147** transmits a signal to the processor and/or transceiver **161** of the BAN. The reader **147,** which is installed in the vicinity of the point of entry, and which has a reliable source of power, can transmit messages to determine if a BAN has entered the vicinity. A BAN that has entered the vicinity of the point of entry receives the signal from the reader **147,** and at **222,** the BAN transmits identification data that identifies the person associated with the BAN. At **223,** the reader receives the transmitted identification data from the BAN, and transmits the identification data associated with the person to the access control management server **110** and/or the digital video management server **120.** At **224,** the access control management server **110** authenticates the person via the identification data, and at **225,** the reader **147** receives a response back from the access management server **110** (and/or the digital video management server **120).** At **226,** the controller **145** operates the door **130** as a function of the response from the access control management server **110,** and/or operates the video sensor **148** as a function of the response from the digital video management server **120.**

It should be understood that there exist implementations of other variations and modifications of the invention and its various aspects, as may be readily apparent, for example, to those of ordinary skill in the art, and that the invention is not limited by specific embodiments described herein. Features and embodiments described above may be combined with each other in different combinations. It is therefore contemplated to cover any and all modifications, variations, combinations or equivalents that fall within the scope of the present invention.

The Abstract is provided to comply with 37C.F.R. §1.72(b) and will allow the reader to quickly ascertain the nature and gist of the technical disclosure. It is submitted with the understanding that it will not be used to interpret or limit the scope or meaning of the claims.

In the foregoing description of the embodiments, various features are grouped together in a single embodiment for the purpose of streamlining the disclosure. This method of disclosure is not to be interpreted as reflecting that the claimed embodiments have more features than are expressly recited in each claim. Rather, as the following claims reflect, inventive subject matter lies in less than all features of a single disclosed embodiment. Thus the following claims are hereby incorporated into the Description of the Embodiments, with each claim standing on its own as a separate example embodiment.

## Claims

1. A system comprising:
a computer processor (160) configured to:
receive body area network (BAN) data (210); and
operate a point of entry as a function of the BAN data (220).

2. The system of claim 1, wherein the computer processor is configured to:
transmit a signal, the signal configured for reception by a BAN, wherein the BAN is associated with a person (221);
receive from the BAN an identification associated with the person (222); and
operate the point of entry as a function of the identification (226).

3. The system of claim 2, wherein the computer processor comprises a server computer processor and a reader computer processor, and wherein:
the reader computer processor is configured to transmit the signal for reception by the BAN (221);
the reader computer processor is configured to receive a response from the BAN, the response comprising the identification associated with the person (222, 223);
the reader computer processor is configured to transmit to the server computer processor the identification associated with the person (223);
the server computer processor is configured to determine an access privilege to the point of entry for the person as a function of the identification of the person (224);
the server computer processor is configured to transmit to the reader computer processor the access privilege to the point of entry for the person (224); and
the reader computer processor is configured to receive the access privilege to the point of entry for the person and operate the point of entry as a function of the access privilege (225, 226).

4. The system of claim 3, wherein the computer processor comprises a controller computer processor (145), and wherein the controller computer processor is coupled to the reader computer processor (147), and the reader computer processor is configured to send a signal to the controller computer processor to operate the point of entry (130) as a function of the access privilege (226).

5. A computer readable medium comprising instruction that when executed by a processor execute a process comprising:
receiving body area network (BAN) data (210); and
operating a point of entry as a function of the BAN data (220).

6. The computer readable medium of claim 5, comprising instructions for:
transmitting a signal, the signal configured for reception by a BAN, wherein the BAN is associated with a person (221);
receiving from the BAN an identification associated with the person (222); and
operating the point of entry as a function of the identification (226).

7. The computer readable medium of claim 6, comprising instructions for:
causing a reader computer processor to transmit the signal for reception by the BAN (221);
causing the reader computer processor to receive a response from the BAN, the response comprising the identification associated with the person (222, 223);
causing the reader computer processor to transmit to a server computer processor the identification associated with the person (223);
causing the server computer processor to determine an access privilege to the point of entry for the person as a function of the identification of the person (224);
causing the server computer processor to transmit to the reader computer processor the access privilege to the point of entry for the person (224); and
causing the reader computer processor to receive the access privilege to the point of entry for the person and operate the point of entry as a function of the access privilege (225, 226).

8. The computer readable medium of claim 7, comprising instructions causing the reader computer processor (147) to transmit a signal to a controller computer processor (145) to operate the point of entry (130) as a function of the access privilege (226).

9. A process comprising:
receiving body area network (BAN) data (210); and
operating a point of entry as a function of the BAN data (220).

10. The process of claim 9, comprising:
transmitting a signal, the signal configured for reception by a BAN, wherein the BAN is associated with a person (221);
receiving from the BAN an identification associated with the person (222); and
operating the point of entry as a function of the identification (226).
